**Europäisches Patentamt**

**European Patent Office**

**Office européen des brevets**

(19)

(11) Veröffentlichungsnummer: **0 027 151**
**A2**

(12) **EUROPÄISCHE PATENTANMELDUNG**

(21) Anmeldenummer: **79104276.5**

(22) Anmeldetag: **02.11.79**

(51) Int. Cl.³: **A 61 B 5/05**
**A 61 N 1/32**

(30) Priorität: **12.10.79 DE 2941371**

(43) Veröffentlichungstag der Anmeldung:
**22.04.81 Patentblatt 81/16**

(84) Benannte Vertragsstaaten:
**AT BE CH FR GB IT LU NL SE**

(71) Anmelder: **Rietz, Erhard**
**Rathsbergstrasse 25**
**D-8500 Nürnberg(DE)**

(72) Erfinder: **Leupold, Karl-Heinz, Dr.h.c.**
**Gudrunstrasse 41**
**D-8500 Nürnberg(DE)**

(74) Vertreter: **Göbel, Matthias**
**Pruppacher Hauptstrasse 5-7**
**D-8501 Pyrbaum-Pruppach(DE)**

(54) **Schaltungsanordnung für eine Vorrichtung zum Messen des Leitwertes menschlicher Organismen.**

(57) Bei der Schaltungsanordnung für eine Vorrichtung zum Messen des Leitwertes menschlicher Organismen und der Durchführung von Auf- und Abbautherapien unter Verwendung von mit einem Meßgerät (7) oder einer regelbaren Spannungsquelle in Verbindung bringbaren Berührungselektroden (8, 9) sind die Berührungselektroden durch Schaltglieder wahlweise mit einer Stromquelle (1) und dem Strommeßgerät (7) in Reihe schaltbar oder mit einer Einrichtung zur Erzeugung einer niederfrequenten Kippschwingungsimpulsspannung regelbarer Größe und verschiedener Polarität verbindbar.

./...

EP 0 027 151 A2

**M. GÖBEL**
PATENT- U. ZIV.-INGENIEUR

**0027151**

**8501 PYRBAUM-PRUPPACH**
PRUPPACHER HAUPTSTRASSE 5-7
TELEFON 09I802 / 675

BANKKONTEN:
VOLKSBANK NÜRNBERG 45233 BLZ 76090000
COMMERZBANK NÜRNBERG 8300907 BLZ 76040061

- 1 -

Firma Erhard Rietz, 8500 Nürnberg

Schaltungsanordnung für eine Vorrichtung zum Messen des Leitwertes menschlicher Organismen

Die Erfindung betrifft eine Schaltungsanordnung für eine Vorrichtung zum Messen des Leitwertes menschlicher Organismen und der Durchführung von Auf- und Abbautherapien unter Verwendung von mit einem Meßgerät oder einer regelbaren Spannungsquelle in Verbindung bringbaren Berührungselektroden.

Vorrichtungen zum Messen von Leitwerten und zur Durchführung von Auf- und Abbautherapien sind als professionelle Laborgeräte bereits bekannt. Sie dienen dazu, durch elektrische Messungen den Gesamtenergiehaushalt zu überwachen bzw. diesen durch die Aufbau- oder Abbautherapie in den Normbereich zu bringen. Die bekannten Vorrichtungen zeichnen sich durch eine komplizierte Ausgestaltung und einen relativ großen Aufbau aus. Außerdem sind sie energieaufwendig.

-./4

Es ist Aufgabe der Erfindung eine einfache Schaltungsanordnung für eine Vorrichtung dieser Art zu schaffen, bei der die Nachteile vermieden sind.

Nach der Erfindung ist dies dadurch erreicht, daß die Berührungselektroden durch Schaltglieder wahlweise mit einer Stromquelle und einem Strommeßgerät in Reihe schaltbar oder mit einer Einrichtung zur Erzeugung einer niederfrequenten Kippschwingungsimpulsspannung regelbarer Größe und verschiedener Polarität verbindbar sind. Zweckmäßig ist die Kippspannung mittels eines Impedanzwandlers und einen durch diesen ansteuerbaren Übertrager gebildet, während der Impedanzwandler selbst durch einen Oszillator ansteuerbar ist, dessen Schaltzeiten mittels eines elektrischen Zeitschaltkreises bestimmbar sind. Die Schaltungsanordnung kann so wirtschaftlich vorteilhaft mit einer geringen Anzahl von Bauteilen auf einer relativ kleinen Schaltungsplatte aufgebracht sein. Bei fehlender Oszillatorspannung sind Impedanzwandler und Verstärker jeweils selbsttätig durch die Betriebsspannung gesperrt, um die Energieabgabe der als Stromquelle wirkenden Batterie gering zu halten.

Weiter ist vorgesehen, die Kippschwingungsimpulsspannungen mittels eines zwischen dem Übertrager und den Berührungs-

-/5

elektroden angeordneten Dreh- oder Schiebewiderstand veränderlich zu machen, so daß die Aufbau- und Abbautherapien mit verschieden großen Intensitäten erfolgen können. Es entspricht der Erfindung, daß die Abbautherapie mit kleiner Intensität bewirkt wird, was vorteilhaft durch eine Einschränkung des Einstellbereichs, z.B. durch Zuordnung eines Festwiderstandes zum Dreh- oder Schiebewiderstand erreichbar ist. Polaritätsänderungen sind schließlich durch einfaches Umkehren der Spannungspotentiale an den Berührungselektroden mittels der Schaltglieder möglich.

Die Erfindung ist anhand eines Schaltbilds verdeutlicht.

Mit 1 ist eine als Stromquelle dienende Batterie von z.B. 9 V bezeichnet, die durch elektrische Leiter 2 und 3 vermittels eines Schalters 4 über einen Regelwiderstand 5 und einen Festwiderstand 6 mit einem Strommeßgerät 7 zur Einregelung eines Basiswertes vor Beginn von Leitwertmessungen, z.B. 100 A, verbindbar ist. Mit 8 und 9 sind zwei unabhängige Berührungselektroden bezeichnet, die zu Leitwertmessungen am menschlichen Organismus durch den Benutzer, etwa zu Hand-Hand-Messungen bzw. Hand-Fuß-Messungen oder Fuß-Fuß-Messungen zu kontaktieren sind. Bei geöffnetem Schalter 4 sind zu Leitwertmessungen die Berührungselektroden 8, 9 mittels eines Schalters 9' mit der Batterie und dem zuvor auf den Basiswert eingeregelten Strommeßgerät 7 in Reihe legbar. Der Anzeigewert am Strommeßgerät 7 gilt als Maß für den energetischen Zustand des Benutzers. Dabei gelten Anzeigewerte zwischen 80 bis 85 $\mu$A als Normwerte. Anzeigewerte kleiner als 80 $\mu$A zeigen die Notwendigkeit einer Aufbautherapie und Anzeigewerte über 85 $\mu$A die Notwendigkeit einer Abbautherapie an.

Zur Aufbau- bzw. Abbautherapie werden durch Drücken der Starttaste 10, die vier parallel geschalteten Kondensatoren 11, 12, 13, 14 auf die Betriebsspannung (9 V) aufgeladen. Am Gatter 15 wird ein Ausgangssignal erzeugt, das am Gatter 16 invertiert und als Freigabesignal den durch die Gatter 17, 18, die Widerstände 19, 20 und den Kondensator 21 gebildeten Oszillator startet. Der Oszillator ist als Bockieroszillator ausgelegt, dessen Widerstände 19, 20 zeitbestimmend wirken. Über einen Widerstand 22 erfolgt die Entladung der Kondensatoren 11 bis 14, wobei die Diode 23 eine Abschalthysterese erzeugt und Abschaltungen mit steiler Flanke bewirkt werden. Das Gatter 18 gibt ein im wesentlichen rechteckförmiges Signal, dessen Amplitude die Betriebsspannung ist. Der Transistor 24 wird von der negativen Flanke des Signals kurzzeitig durchgeschaltet, wobei die Schaltzeiten durch den Widerstand 25 und den Kondensator 26 bestimmt sind. Mit 27 ist ein als Impedanzwandler wirkender weiterer Transistor bezeichnet, der einen Übertrager 28 ansteuert. Dem Transistor 27 ist ein Kompensationskondensator 29 zugeordnet, der auch als Dämpfungsglied für die Ein- bzw. Ausschwingvorgänge wirkt. Die Transistoren 24, 27 sind bei fehlender Oszillatorspannung jeweils selbsttätig durch die Betriebsspannung gesperrt, was sich stark stromsparend für die Schaltungsanordnung auswirkt.

In der gezeigten oberen Stellung des Schalters 30 sind über den veränderbaren Widerstand 31 zu Aufbautherapien Spannungen zwischen 0 und 30 V auf die Berührungselektroden, 8, 9 legbar.

Zu Aufbautherapien, die mit kleiner Spannungsamplitude bewirkt werden sollen, ist der Schalter 30 in die untere Schaltstellung zu bewegen. Hierbei wird den Berührungselektroden 8, 9 ein den Einstellbereich einschränkender Widerstand 32 dem Widerstand 31 vorgeschaltet und gleichzeitig die Polarität der Berührungselektroden 8, 9 umgekehrt.

Die Abbautherapien können so mit kleinen Intensitäten erfolgen, wobei gesichert ist, daß Spannungsamplituden von 30 V nicht erreicht werden.

Zur Vermeidung einer Zerstörung der Batterie 1 bei Falschanklemmung ist eine Diode 33 und als Batteriepuffer ein Kondensator 34 vorgesehen.

Die Schaltungsanordnung zeichnet sich durch wenige Bauteile und kleinen Abmessungen aus. Sie ist gleichzeitig stromsparend ausgelegt, was für einen mobilen Einsatz der Vorrichtung entscheidend ist.

# M. GÖBEL
### PATENT- U. ZIV.-INGENIEUR

**8501 PYRBAUM-PRUPPACH**
PRUPPACHER HAUPTSTRASSE 5-7
TELEFON 09180 2 / 675

BANKKONTEN:
VOLKSBANK NÜRNBERG 452 33 BLZ 760 900 00
COMMERZBANK NÜRNBERG 8 300 907 BLZ 760 400 61

**0027151**

29. Okt. 1979

Firma Erhard Rietz, 8500 Nürnberg

-7-

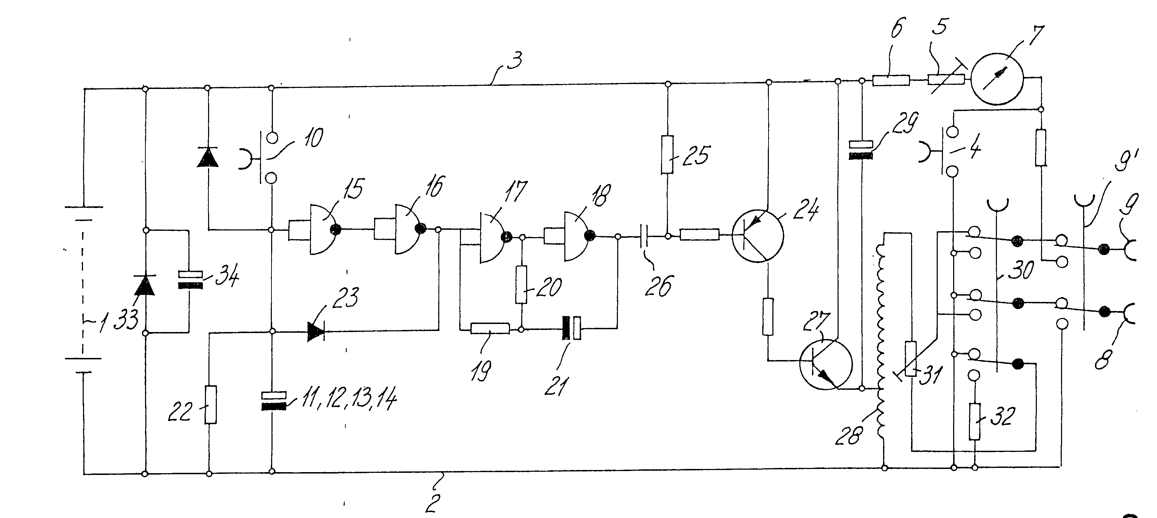

Patentansprüche

1. Schaltungsanordnung für eine Vorrichtung zum Messen des Leitwertes menschlicher Organismen und der Durchführung von Auf- und Abbautherapien unter Verwendung von mit einem Meßgerät oder einer regelbaren Spannungsquelle in Verbindung bringbaren Berührungselektroden, dadurch gekennzeichnet, daß die Berührungselektroden (8, 9) durch Schaltglieder wahlweise mit einer Stromquelle (1) und einem Strommeßgerät (7) in Reihe schaltbar oder mit einer Einrichtung zur Erzeugung einer niederfrequenten Kippschwingungsimpulsspannung regelbarer Größe und verschiedener Polarität verbindbar sind.

2. Schaltungsanordnung nach Anspruch 1, dadurch gekennzeichnet, daß die Kippschwingungsimpulsspannung mittels eines Impedanzwandlers und eines durch diesen ansteuerbaren Übertragers gebildet ist.

-/2

3. Schaltungsanordnung nach Anspruch 1 oder 2, dadurch gekennzeichnet, daß der Impedanzwandler durch einen Oszillator ansteuerbar ist und daß die Schaltzeiten des Impedanzwandlers mittels eines elektrischen Zeitschaltkreises bestimmbar sind.

4. Schaltungsanordnung nach Anspruch 1, dadurch gekennzeichnet, daß die Kippschwingungsimpulsspannungen mittels eines zwischen dem Übertrager (28) und den Berührungselektroden (8, 9) angeordneten Dreh- oder Schiebewiderstands veränderlich sind.

5. Schaltungsanordnung nach Anspruch 4, dadurch gekennzeichnet, daß der Einstellbereich der Kippschwingungsimpulsspannungen mittels eines zum Dreh- oder Schiebewiderstand zuschaltbaren Widerstands verringerbar ist.

0027151